# EUROPEAN PATENT SPECIFICATION

(11) **EP 0 747 090 B1**
(45) Date of publication and mention of the grant of the patent: **24.07.2002**
(21) Application number: 96302999.6
(22) Date of filing: 29.04.1996
(51) Int. Cl.: A61M 39/28, A61M 39/08, F16K 7/06

(54) **Fluid flow control device, in particular for infusing intravenous fluids**
Vorrichtung zur Durchflusskontrolle von Flüssigkeiten, insbesondere zur Infusion von intravenösen Flüssigkeiten
Dispositif de contrôle du débit des fluides, en particulier pour perfuser des liquides intraveineux

(30) Priority: 11.05.1995 GB 9509546
(43) Date of publication of application: 11.12.1996
(73) Proprietor: The BOC Group plc, Windlesham Surrey GU20 6HJ (GB)
(72) Inventor: Karlberg, Rolf Emil, 246 33 Loddekopinge (SE)
(74) Representative: Ruffles, Graham Keith

(56) References cited:
- WO-A-85/02664
- US-A- 4 974 811

## Description

The present invention relates to devices for fluid flow control and, in particular, to such devices when forming part of a system for infusing fluids into the body of a patient.

It is known to introduce intravenous fluids such as blood, plasma or drugs into a patient by means of a hollow needle connected by tubing to an elevated bottle or bag forming a reservoir for the fluid. The elevated bottle or bag provides a gravity feed into the patient's vein. Usually, a small pinch valve is used to control the flow rate of the fluid through the tubing from the bottle or bag to the patient.

This known method is acceptable in many cases where flow rates are not critical. However, it does suffer from the disadvantage that the flow rate can vary widely due to the elevation of the bag or bottle, the liquid level within the bag or bottle and the temperature and hence viscosity of the fluid in liquid phase. A further disadvantage which will vary the flow rate is the instability of tubing when pinched.

For some types of treatment the rate of drug injection is critical since excess doses can be toxic.

In International Patent Publication Number WO85/02664, there is described a resilient tube of triangular transverse cross-section which is used for transporting parenteral fluid for delivery to a patient. The tube extends through a device which includes a housing and a roller. The roller is used to squeeze the tube for regulating the flow of fluid therethrough.

Force is applied by the roller against an apex of the triangular tube forcing said apex towards the opposite side of the triangle.

This document does address the problem of controlling accurately the flow of liquid through a resilient tube. However, the solution offered in this document does not give the same degree of resolution as that of the present invention.

It is an aim of the present invention to provide a fluid flow control device which is simple to manufacture, easy to operate and which is capable of accurately controlling the flow rate of a fluid through a tube.

According to the present invention, a fluid flow control device comprises a resilient hollow tube having a passage with an internal cross-sectional configuration defined in part by two sides meeting at an apex for the flow therethrough of a fluid and means for applying a pressure to the tube to reduce in a controlled manner the cross-sectional area of the passage, characterised in that the means is located adjacent the tube opposite said apex and movable towards said apex to apply a pressure to the tube.

In a preferred embodiment, the internal cross-sectional configuration is defined by a three-sided passage, the pressure means being aligned with one side of the passage for movement towards the opposite apex.
Preferably the passage has a cross-sectional configuration in the form of a straight-sided equilateral triangle.

The tube may be made from silicone.

Preferably, the resilient hollow tube is located within a housing, which is provided at a location between the ends of the resilient hollow tube with a through aperture within which a steel ball is movable between a first position in which the ball projects into the housing and maintains a portion of the tube in a compressed closed condition and a second position in which the portion is substantially unaffected by the ball.

An embodiment of the invention will now be described, by way of example, reference being made to the Figures of the accompanying diagrammatic drawings in which:-
Figure 1 is a longitudinal cross-section through a fluid flow control device illustrating the position of certain parts when there is no restriction against flow of fluids through a passage in a tube forming part of the device;
Figure 2 is a cross-section similar to Figure 1 illustrating the position of certain parts when a restriction has been applied to the passage;
Figure 3 is a cross-section similar to Figures 1 and 2 illustrating the position of certain parts when the flow of fluid through the passage is stopped;
Figure 4 is a cross-section through the device illustrated in Figure 1;
Figures 5a, 5b and 5c are cross-sections similar to Figure 4 but illustrating the cross-sectional configuration of the passage for varying positions of a pressure means;
Figures 6a, 6b and 6c are cross-sections similar to Figures 5a, 5b and 5c but showing the cross-sectional configuration of a normally circular cross-sectional passage for equivalent positions of the pressure means;
Figure 7 is a cross-section through an alternative tube; and
Figure 8 is a cross-section through yet a further tube.

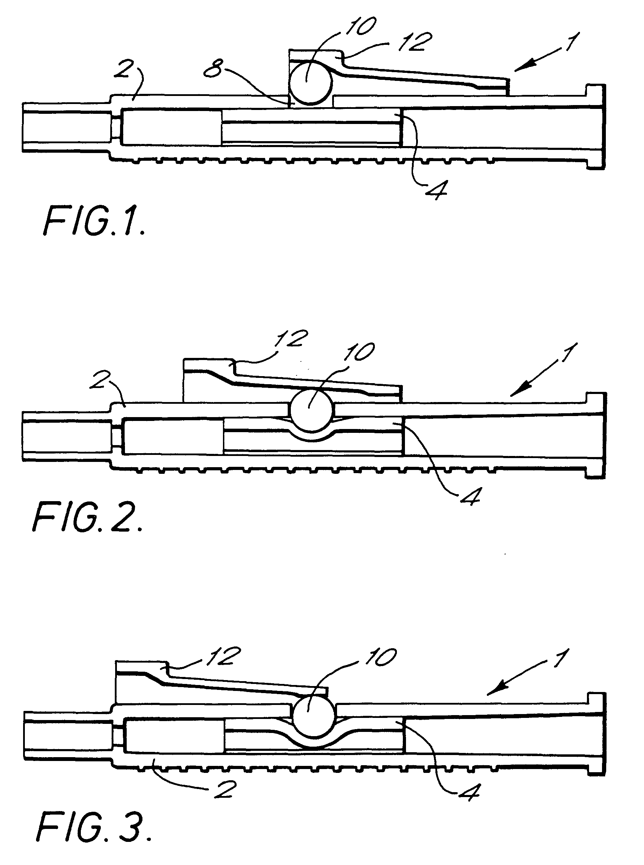

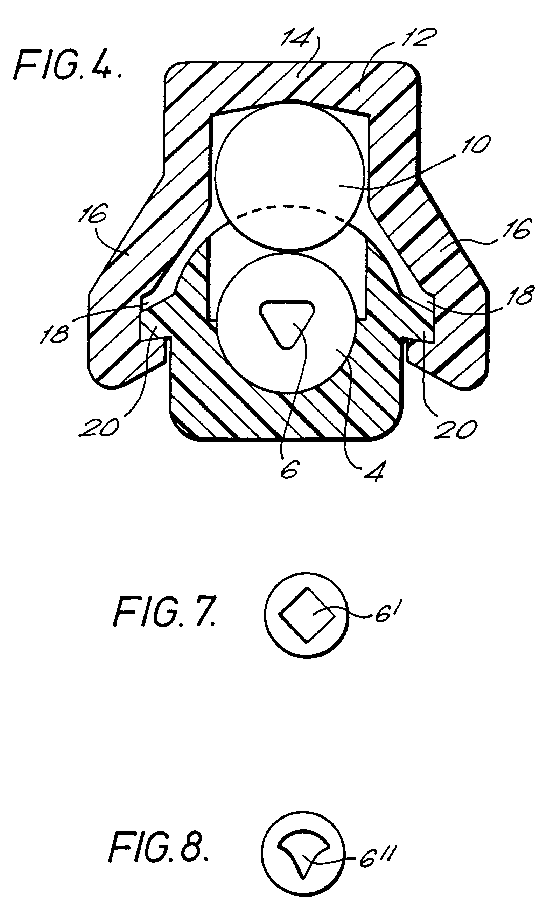

As shown in Figures 1 to 4, a fluid flow control device 1 includes a tubular housing 2 made for example of polypropylene which forms part of a system for infusing liquids into the body of a patient. Affixed within the housing 2 is a resilient hollow tube 4 made, for example, of silicone. As shown most clearly in Figure 4, the internal cross-sectional configuration of the tube 4 is defined by a three-sided passage 6. A through aperture 8 is formed in the housing 2 approximately mid-way between the ends of the tube 4. Located within the aperture 8 is pressure means in the form of a steel ball 10. It is important to note that the ball 10 is aligned with one side (as shown the topside) of the three-sided passage 6 opposite one apex.

A slide 12 is mounted on the upper (as shown) surface of the housing 2 and is capable of movement longitudinally of the housing 2. The slide 12 has an inverted U-shape with a web 14 and two depending flanges 16. Each flange 16 includes on its inside surface, a channel 18 adapted to receive a runner 20 formed on the surface of the housing 2. The inside surface of the web 14 is inclined as shown in Figures 1 to 3 and engages the upper (as shown) surface of the ball 10.

In use, when the tubular housing 2 forms part of a system for infusing a liquid drug into a patient said liquid will flow through the housing 2 and hence through the three-sided passage 6 in the tube 4. According to the particular clinical condition of the patient and the nature of the liquid with which the patient is being treated, the flow rate through the housing 2 may need to be varied from as little as 5ml per hour to as much as 1000ml per hour.

As shown in Figure 1, with the slide 12 in the position illustrated, the ball 10 will apply substantially no pressure to the surface of the tube 4 and the passage 6 will not be occluded. This will allow maximum flow of liquid through the housing 2.

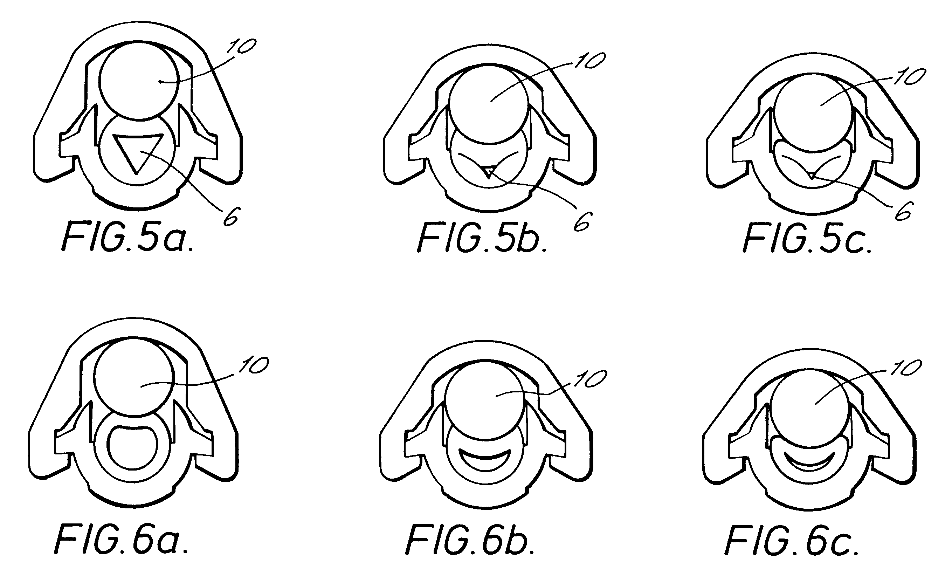

If it is desired to reduce the flow rate of the fluid flowing through the housing 2, then the slide 12 is moved to the left as seen in Figure 2. The inclined inner surface of the web 14 will urge the ball 10 inwardly through the aperture 8 towards one of the apices to compress the tube 4. This will have the effect of distorting the triangular cross-section of the passage 6 and thereby reduce the flow rate of fluid through said passage 6 (see Figures 5a, 5b and 5c).

Figure 3 illustrates the position of the slide 12 and ball 10 when the passage 6 in the tube 4 is completely occluded which prevents the flow of any fluid through the housing 2.

Turning now to Figures 5a, 5b and 5c these illustrate the varying cross-sectional configurations of the passage 6 for varying pressures to the tube 4 applied by the ball 10.

It has been found that by using a three-sided passage 6 in the resilient hollow tube 4 and employing a closing pressure to one side of the three-sided passage in a direction towards an apex, then a high resolution giving accurate changes in the rate of flow of fluid through the passage 6 can be achieved.

Figures 6a, 6b and 6c illustrate the manner in which the cross-sectional configuration of a standard circular cross-sectioned tube would alter for the same pressures applied by the ball 10 as per Figures 5a, 5b and 5c. The shape of the passage as it is deformed makes it far more difficult to control the rate of change of fluid passing through the passage as compared to a three-sided passage.

It has been found that good resolution of fluid flow through a passage 6',6" can be obtained with cross-sectional configurations as illustrated in Figures 7 and 8. The common feature is the passage 6, 6' or 6" being defined in part by two sides meeting at an apex and the pressure means eg. the ball 10 being directed towards the apex when squeezing or occluding the passage 6, 6' or 6".

Although in the above described embodiment the three-sided passage 6 is shown with straight sides and radiused apices in a preferred embodiment the passage is in the form of an equilateral triangle with sharp corners.

Alternatively, the sides can be made convex or concave.

## Claims

1. A fluid flow control device (1) comprising a resilient hollow tube (4) having a passage (6) with an internal cross-sectional configuration defined in part by two sides meeting at an apex for the flow therethrough of a fluid and means for applying a pressure to the tube (4) to reduce in a controlled manner the cross-sectional area of the passage (6), **characterised in that** the means (10) is located adjacent the tube (4) opposite said apex and movable towards said apex to apply a pressure to the tube.

2. A fluid flow control device as claimed in Claim 1, in which the internal cross-sectional configuration is defined by a three-sided passage, the pressure means being aligned with one side of the passage for movement towards the opposite apex.

3. A fluid flow control device as claimed in Claim 2, in which the passage has a cross-sectional configuration in the form of a straight-sided triangle.

4. A device as claimed in Claim 3, in which the straight-sided triangle is an equilateral triangle.

5. A device as claimed in any one of Claims 1 to 4, in which the tube is made from silicone.

6. A fluid flow control device as claimed in any one of Claims 1 to 5, in which the resilient hollow tube is located within a housing (2) which is provided at a location between the ends of the hollow resilient tube with a through aperture within which said pressure means is movable between a first position in which the means projects into the housing and maintains a portion of the tube in a compressed closed condition and a second position in which the portion is substantially unaffected by the pressure means.

7. A fluid flow control device as claimed in Claim 6, in which a slide (12) is mounted on a surface of the housing and is movable in a longitudinal direction of the housing and includes a surface for engaging and moving the pressure means between said first and second positions.

8. A fluid flow control device as claimed in Claim 6 or 7, in which the pressure means is a steel ball.

9. In a system for infusing fluids into the body of a patient, a fluid flow control device as claimed in any one of claims 1 to 8.

## Patentansprüche

1. Fluidfluß-Regelungsvorrichtung (1), die folgendes aufweist: einen elastischen, hohlen Schlauch (4), der einen Durchgang (6) mit einer Querschnitt-Innenkonfiguration, die teilweise durch zwei Seiten definiert wird, die sich an einem Scheitelpunkt treffen, für den Fluß eines Fluids durch diesen hat, und ein Mittel zur Ausübung eines Drucks auf den Schlauch (4), um die Querschnittfläche des Durchgangs (6) auf kontrollierte Weise zu verringern, **dadurch gekennzeichnet, daß** sich das Mittel (10) anschließend an den Schlauch (4) gegenüber dem Scheitelpunkt befindet und zu dem Scheitelpunkt hin bewegt werden kann, um einen Druck auf den Schlauch auszuüben.

2. Fluidfluß-Regelungsvorrichtung nach Anspruch 1, bei welcher die Querschnitt-Innenkonfiguration durch einen dreiseitigen Durchgang definiert ist, wobei das Druckmittel mit einer Seite des Durchgangs zur Bewegung hin zu dem gegenüberliegenden Scheitelpunkt ausgerichtet ist.

3. Fluidfluß-Regelungsvorrichtung nach Anspruch 2, bei welcher der Durchgang eine Querschnittkonfiguration in Form eines geradseitigen Dreiecks hat.

4. Vorrichtung nach Anspruch 3, bei der das geradseitige Dreieck ein gleichseitiges Dreieck ist.

5. Vorrichtung nach einem der Ansprüche 1 bis 4, bei welcher der Schlauch aus Silicon hergestellt wird.

6. Fluidfluß-Regelungsvorrichtung nach einem der Ansprüche 1 bis 5, bei der sich der elastische, hohle Schlauch innerhalb eines Gehäuses (2) befindet, das an einer Stelle zwischen den Enden des hohlen, elastischen Schlauches mit einer Durchgangsöffnung versehen ist, innerhalb der das Druckmittel zwischen einer ersten Position, in der das Mittel in das Gehäuse hinein vorsteht und einen Abschnitt des Schlauches in einem zusammengedrückten, geschlossenen Zustand hält, und einer zweiten Position bewegt werden kann, in welcher der Abschnitt durch das Druckmittel im wesentlichen nicht beeinflußt wird.

7. Fluidfluß-Regelungsvorrichtung nach Anspruch 6, bei der auf einer Oberfläche des Gehäuses ein Schieber (12) angebracht ist und in einer Längsrichtung des Gehäuses bewegt werden kann und der eine Oberfläche für das Eingreifen in das Druckmittel und dessen Bewegung zwischen der ersten und zweiten Position einschließt.

8. Fluidfluß-Regelungsvorrichtung nach Anspruch 6 oder 7, bei der das Druckmittel eine Stahlkugel ist.

9. In einem System zum Infundieren von Fluids in den Körper eines Patienten eine Fluidfluß-Regelungsvorrichtung nach einem der Ansprüche 1 bis 8.

## Revendications

1. Dispositif de régulation de l'écoulement de fluides (1) comprenant un tube élastique creux (4) comportant un passage (6) ayant une configuration de section transversale interne définie en partie par deux côtés se rencontrant au niveau d'un sommet, pour permettre l'écoulement d'un fluide à travers celui-ci, et un moyen pour appliquer une pression au tube (4) pour réduire de manière contrôlée la surface de section transversale du passage (6), **caractérisé en ce que** le moyen (10) est agencé près du tube (4) en un point opposé audit sommet et peut être déplacé vers ledit sommet pour appliquer une pression au tube.

2. Dispositif de régulation de l'écoulement de fluides selon la revendication 1, dans lequel la configuration de la section transversale interne est définie par un passage à trois côtés, le moyen de pression étant aligné avec un côté du passage en vue d'un déplacement vers le sommet opposé.

3. Dispositif de régulation de l'écoulement de fluides selon la revendication 2, dans lequel le passage a une configuration de la section transversale en forme d'un triangle à côtés droits.

4. Dispositif selon la revendication 3, dans lequel le triangle à côtés droits est un triangle équilatéral.

5. Dispositif selon l'une quelconque des revendications 1 à 4, dans lequel le tube est composé de silicone.

6. Dispositif de régulation de l'écoulement de fluides selon l'une quelconque des revendications 1 à 5, dans lequel le tube élastique creux est agencé dans un boîtier (2) agencé au niveau d'un emplacement situé entre les extrémités du tube élastique creux, et comportant une ouverture de passage dans laquelle ledit moyen de pression peut être déplacé entre une première position, dans laquelle le moyen déborde dans le boîtier et maintient une partie du tube dans un état comprimé fermé, et une deuxième position dans laquelle la partie correspondante n'est pratiquement pas affectée par le moyen de pression.

7. Dispositif de régulation de l'écoulement de fluides selon la revendication 6, dans lequel un coulisseau (12) est monté sur une surface du boîtier et peut être déplacé dans une direction longitudinale par rapport au boîtier et englobe une surface destinée à s'engager dans le moyen de pression et à le déplacer entre lesdites première et deuxième positions.

8. Dispositif de régulation de l'écoulement d'un fluide selon les revendications 6 ou 7, dans lequel le moyen de pression est une bille en acier.

9. Dispositif de régulation de l'écoulement d'un fluide selon l'une quelconque des revendications 1 à 8, utilisé dans un système de perfusion de fluides dans l'organisme d'un patient.
